# EUROPEAN PATENT APPLICATION

(11) **EP 0 639 375 A1**
(43) Date of publication of application: **22.02.1995**
(21) Application number: 94610043.5
(22) Date of filing: 12.08.1994
(51) Int. Cl.: A61K 31/195, A61K 9/08

(54) **A bile duct deterging composition**

(30) Priority: 16.08.1993 JP 235986/93
(71) Applicant: Senju Pharmaceutical Co., Ltd., Osaka-shi, Osaka 541 (JP)
(72) Inventor: Moriyasu, Akihito, Sendai-shi, Miyagi 981-31 (JP)
(74) Representative: Plougmann, Vingtoft & Partners A/S

(57) **Abstract**

A bile duct deterging composition which comprises from about 10 w/v% to about 40 w/v% of N-acetylcysteine as an effective component or an alkali metal salt thereof and which is an aqueous solution adjusted to a pH of 7.0 to 8.0.

## Description

### BACKGROUND OF THE INVENTION

### 1. Field of the Invention

The present invention relates to a bile duct deterging composition and use thereof. More particularly, the invention relates to a bile duct deterging composition containing N-acetylcysteine primarily used in detailed examination and observation of the bile duct using cholangioscopy or cholangiography (both are generically referred to as detailed examination of the bile duct).

### 2. Detailed Description of the Related Art

The bile duct or biliary tract generally refers to a passage through which bile produced in the liver is transported to the duodenum. Specifically, the bile duct refers to a series of bile excretory routes that form the bilateral hepatic duct by the convergence of the intra-hepatic bile ductsin the liver, the common hepatic duct, the cystic duct carrying bile to and from the gallbladder, and also to the gallbladder storing the bile in the midway.

In the adult, about 500 ml to 1500ml of bile is normally excreted per day to the duodenum via the bile duct. When the bile duct is obstructed because of exclusion caused by bile duct cancer and other kinds of cancers involving surrounding organs, cholestasis is generated in the bile duct with the result that the cholestasis promotes the precipitation and agglutination of the mucus of the bile duct and calcium bilirubinate. Moreover, with the bile infection from the duodenum being added because of cholestasis, biliary sludge and gallstones are produced in the bile duct. In addition, a complete obstruction of the bile duct produces obstructive jaundice.

To release cholestasis and obstruction of the bile duct, doctors perform percutaneous transhepatic biliary drainage (hereinafter simply referred to PTBD). A contrast medium can be injected directly into the bile duct to capture an image of the bile duct, which image forms a basis for diagnosing a pathological change in the bile duct such as gallstones and cancer which cause cholestasis and bile duct obstruction. Along with the development of optical apparatuses including a cholangioscopy, cases are increasing in recent years in which the PTBD-route is expanded to allow the cholangioscopy to be inserted therethrough for a detailed examination and observation of the bile duct thereby enabling diagnosis and treatment of pathological change in the bile duct with respect to cancer and gallstones which cause cholestasis and bile duct obstruction.

However, when cholestasis occurs, direct cholangiography cannot provide a sufficient image of the biliary tree. In addition, even the use of cholangioscopy fails to allow doctors to examine in detail and to observe a pathological change in the bile duct (such as cancer and gallstones). Thus a problem arises that accurate diagnosis cannot be carried out very often. Conventionally, an injection of saline solution has been followed by a detailed examination and observation of the bile duct. Such procedure cannot provide a sufficient advantage because substances in the bile duct, mainly comprising of mucus cannot be removed.

On the other hand, gallstones roughly fall into two categories: cholesterol gallstones and pigment gallstones. N-acetylcysteine is known as an agent which promotes the disintegration of cholesterol gallstones. The action is so weak that N-acetylcysteine must be forced to contact gallstones for about ten hours to several weeks (Scand. J. Gastroent et. al, 24, 373-380 (1989); Gastroenterology., 98, 454-463 (1990)). To solve the above problem, the inventors of the present invention have proposed a combined use of N-acetylcyteine with alkali metal bicarbonate for effectively disintegrating gallstones (see European Patent Application No. 0497724A1).

### SUMMARY OF THE INVENTION

The present invention provides a bile duct deterging composition which comprises about 10 w/v% to 40 w/v% of N-acetylcysteine as an effective component or an alkali metal salt thereof, which composition is an aqueous solution adjusted to a pH of 7.0 to 8.0.

The deterging composition of the present invention neither substantially disintegrates nor dissolves gallstones produced in the bile duct, but can remove biliary substances including biliary sludges which are mainly comprised of mucus that adheres to the bile duct wall. Therefore, the deterging composition is primarily used in the diagnosis (detailed examination and observation) of the bile duct and in the treatment of the bile duct and related organs. The composition is very useful in the easy and accurate diagnosis of pathological change in the bile duct.

### DESCRIPTION OF THE PREFERRED EMBODIMENTS

A bile duct deterging composition of the present invention is an aqueous solution comprising N-acetylcysteine as an effective component or an alkali metal salt (for example, sodium salt or potassium salt) thereof. The concentration of the effective component in the aqueous solution ranges from about 10 to 40% both inclusive (% refers to w/v % unless otherwise indicated), or preferably from about 10 to 30% both inclusive, or more preferably from about 15 to 25% both inclusive such as, for example, 15%, 20% or 25%. The pH of the aqueous solution is adjusted to from about 7.0 to 8.0, preferably from, 7.5 to 8.0 such as, for example, 7.5, 7.6, 7.7, 7.8, 7.9 and 8.0. Physiologically acceptable bases such as sodium hydrogen carbonate, potassium hydrogen carbonate, sodium hydroxide and potassium hydroxide can be used for this purpose. Among them, sodium hydrogen carbonate is particularly preferably used.

The bile duct deterging composition of the present invention (hereinafter simply referred to as the deterging composition) may contain various additives such as a buffering agent, an isotonic agent, an preservative and an antibiotic that can be used in the preparation of injections or infusions. Examples of preservatives that can be used here include quaternary ammonium salts (such as benzalkonium chloride), Parabens (such as methyl or ethyl ester of parahydroxybenzoic acid) and chlorobuthanol. Additionally, another compound may be contained which is capable of deterging the bile duct depending on the situation.

The deterging composition can be prepared by dissolving a predetermined amount of the effective component in water, preferably in distilled or sterilized water, followed by adding a pH adjusting agent to adjust the pH of the deterging composition.

Alternatively, the deterging composition can be prepared by dissolving predetermined amounts of the effective component and the pH adjusting agent in water.

The deterging composition of the present invention can be used in a method for deterging the bile duct. Specifically, the deterging composition is preferably injected in the same amount as the bile duct capacity into the bile duct of a patient (suspected of bile duct cancer or cancers involving organs surrounding the gallbladder or suspected of bile duct diseases caused by gallstones) followed by holding the state for a predetermined time to clean the bile duct. The cleaned bile duct of the patient can be then diagnosed by cholangiography, such as X-ray photograph using a contrast medium in the bile duct or by direct observation with cholangioscopy. Examples of the contrast medium include iopamidol and meglumine sodium amidotrizoate.

In the above method, the deterging compositions can be injected via a percutaneous and intrahepatic tube or a tube provided in the bile duct. The deterging composition can be held in the bile duct through the clamping of the above tube.

The amount of the deterging composition to be used varies on the concentration of the effective component in the deterging composition, and the age, weight, and condition of the patient. For example, an aqueous solution containing N-acetylcysteine is used in the same amount as the capacity of the bile duct (normally 10 to 20ml in adults). The deterging composition is held in the bile duct usually for about 20 to 30 minutes. When such one-time administration of the bile duct deterging composition proves to be insufficient, a plurality of times administration thereof, namely two to ten times administration and holding the deterging composition in the bile duct can be repeated.

Moreover, in the repeated administration to the bile duct, the discharge of the bile duct deterging composition from the bile duct is desirably conducted by using a saline solution (in the same as the amount or more of the used deterging composition).

### EXAMPLE

Example 1 Bile Duct Deterging Composition

A bile duct deterging composition was formulated by the conventional method in accordance with the following prescription.

| | |
|---|---|
| N-Acetylcysteine | 1.762g |
| (as sodium acetylcysteine | 2.000g) |
| Sodium hydrogen carbonate | 0.952g |

The total amount of the deterging composition was set to 10ml by addition of purified and sterilized water. An ampoule was filled with the resulting solution to provide a bile duct deterging composition.

### [Experiment Example 1]

Using the method of Ueda et al. reported in the Japanese Society of Gastroenterological Surgery Vol. 85 No. 9, page 1724 (1988), a model of cholestasis and a model of bile duct infection were prepared.

A hybrid adult dog which weighed 15Kg was used in an experiment in which a tube with an external diameter (1mm) was inserted into the branch of the bile duct that is drainaged from the second segment of the left lobe of the liver whereas the end of the tube leading to the duodenum was closed. This resulted in the formation of the state of cholestasis in the partial liver. Furthermore, 10⁷ E. Coli and 10⁷ Bacteroides Frangilis that had been clinically separated were simultaneously injected so that the model of cholestasis and the model of bile duct infection were prepared with the dog after three months.

10ml of 20% bile duct deterging composition which had been formulated in Example 1 was injected via the tube that had been installed as described into the bile duct of the dog in which the model of cholestasis was prepared. Images of the bile duct were captured with the cholangioscopy before the injection of the above deterging composition and 30 minutes after. Before the injection of the deterging composition, bile duct substances such as mucus and biliary sludges occupied the bile duct in mixture with gallstones. Thus, it had been confirmed that the nature of the bile duct wall and the location of the gallstones can hardly be observed with clarity. In contrast, when the image of the bile duct was captured by injecting the deterging composition, the mucus and biliary sludges were removed by the dissociation and removal of the bile duct substances by the deterging composition, thereby enabling detailed observation of the terminal bile duct.

Since the bile duct deterging composition according to the present invention has an excellent action of dissociating and removing bile duct substances, it can be favorably used in combination with the detailed examination of the biliary tract. Furthermore, according to the method for deterging the bile duct of the present invention, the bile duct can be clearly examined and observed at the time of the detailed examination of the bile duct.

With respect to two cases in which informed consent was given, the deterging composition was used for deterging the bile duct and examining the bile duct in detail.

### Case 1: 44-Year-Old Female Patient Contracted Hepatolithiasis (In Detailed Examination Before the Operation)

Since the above female patient suspected of hepatolithiasis was filled with calculi, she was subjected to retrograde cholangiography such as ERCP. As a result of the examination, even the presence of the calculus was not detected, let alone the location of the calculus. Under these circumstances, a PTBD-tube was penetrated to inject 6ml/day of 20% N-acetylcysteine solution. As a result of three administrations thereof, calculus was present in the bile duct only, which produces a space with the result that the passage to the center became favorable and all the portions of the intra-hepatic bile duct including the bile duct filled with calculi were subjected to cholangiography, which was extremely useful for detailed examination.

### Case 2: A 85-Year Old Female Patient Contracted Choledocholithiasis

The above female patient was diagnosed as being unable to undergo operation because of old age and had her calculi were removed with cholangioscopy. However, the calculi were buried in the bile duct substance, so that it was difficult to identify them. For deterging the bile duct, 18ml of 20% N-acetylcysteine solution was injected three times from percutaneous transhepatic cholangioscopy (PTCS) tube. As a consequence, only gallstones remained in the bile duct and they could be clearly identified with the cholangioscopy.

## Claims

1. A bile duct deterging composition which comprises from about 10 w/v% to about 40 w/v% of N-acetylcysteine as an effective component or an alkali metal salt thereof, and which is an aqueous solution adjusted to a pH of 7.0 to 8.0.

2. A composition according to claim 1, wherein the pH is adjusted by using sodium hydrogen carbonate.

3. A composition according to claim 1, wherein N-acetylcysteine or the alkali metal salt thereof has a concentration of about 15 w/v% to about 25 w/v%.

4. A composition according to claim 1, wherein the alkali metal salt of N-acetylcysteine is sodium salt.

5. Use of N-acetylcysteine or an alkali metal thereof for the preparation of a bile duct deterging composition.
